# EUROPEAN PATENT SPECIFICATION

(11) **EP 0 535 104 B1**
(45) Date of publication and mention of the grant of the patent: **04.09.1996**
(21) Application number: 91911908.1
(22) Date of filing: 21.06.1991
(51) Int. Cl.: C07D 487/04, A61K 31/495

(54) **HETEROCYCLIC COMPOUNDS AND THEIR PREPARATION AND USE**
HETEROCYCLISCHE VERBINDUNGEN, HERSTELLUNG UND VERWENDUNG
COMPOSES HETEROCYCLIQUES, LEUR PREPARATION ET UTILISATION

(30) Priority: 22.06.1990 DK 1518/90
(43) Date of publication of application: 07.04.1993
(73) Proprietor: NOVO NORDISK A/S, 2880 Bagsvaerd (DK)
(72) Inventor: HANSEN, Holger, Claus, DK-3500 V rl se (DK)
(86) International application number: DK9100170
(87) International publication number: WO9200298

(56) References cited:
- EP-A- 0 225 013
- EP-A- 0 226 282
- EP-A- 0 283 162
- EP-A- 0 320 136
- EP-A- 0 344 943
- EP-A- 0 347 094

## Description

The present invention relates to therapeutically active heterocyclic compounds, a method of preparing the same, pharmaceutical compositions comprising the compounds, and to methods of treating therewith. The novel compounds are useful in psychopharmaceutical applications, e.g., in the treatment of central nervous system ailments, for example, as anticonvulsants or anxiolytics, hypnotics, in treating emesis, schizophrenia, or in improving the cognitive function of the brain.

It is well known (Squires, R.F. and Braestrup, C. in Nature (London) 266 (1977) 732-734) that specific sites in the central nervous systems of vertebrates exhibit a high specific affinity for binding 1,4- and 1,5-benzodiazepines. These sites are called benzodiazepine receptors.

It has now been found that members of a novel group of aminoimidazoquinoxaline and -quinazoline compounds have strong affinity for the benzodiazepine receptors which make them useful in psychopharmaceutical preparations.

Accordingly, it is an object of the invention to provide such novel 4-aminoimidazoquinoxaline and 5-aminoimidazoquinazoline compounds.

The compounds of the invention have the general formula I 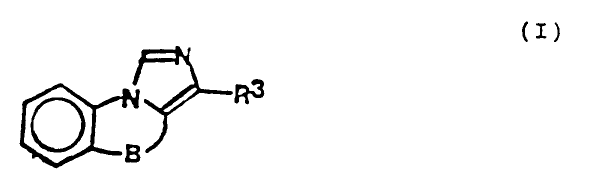 wherein
R³ is 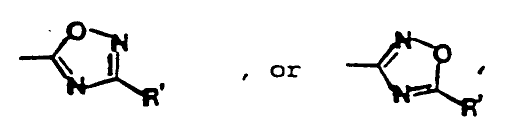 wherein R' is H, C₁₋₆-alkyl or
C₃₋₇-cycloalkyl;
-B- is -C(R'')=N- or -N=C(R'')-
wherein R'' is -NR'''R'''',
wherein R''' and R'''' independently are H, C₁₋₆-alkoxy, C₃₋₇-cycloalkyl or C₁₋₆-alkyl.

Specifically, the invention is related to the following compounds:
3-(5-cyclopropyl-1,2,4-oxadiazol-3-yl)-4-dimethylamino-imidazo[1,5-a]quinoxaline hydrochloride.
3-(5-cyclopropyl-1,2,4-oxadiazol-3-yl)-5-dimethylamino-imidazo[1,5-a]quinoxaline.
3-(3-cyclopropyl-1,2,4-oxadiazol-5-yl)-5-morpholino-imidazo[1,5-a]quinazoline.
3-(5-cyclopropyl-1,2,4-oxadiazol-3-yl)-5-morpholino-imidazo[1,5-a]quinazoline.
3-(5-cyclopropyl-1,2,4-oxadiazol-3-yl)-4-morpholino-imidazo[1,5-a]quinoxaline.
3-(5-cyclopropyl-1,2,4-oxadiazol-3-yl)-5-thiomorpholino-imidazo[1,5-a]quinazoline.
3-(3-cyclopropyl-1,2,4-oxadiazol-5-yl)-5-thiomorpholino-imidazo[1,5-a]quinazoline.
6-chloro-3-(3-cyclopropyl-1,2,4-oxadiazol-5-yl)-5-morpholino-imidazo[1,5-a]quinazoline.
ethyl 5-morpholino-imidazo[1,5-a]quinazoline-3-carboxylate.
ethyl-6-chloro-5-morpholino-imidazo[1,5-a]quinazoline-3-carboxylate.

The invention also relates to a method of preparing the above mentioned compounds. This method comprises:
a) reacting a compound of formula II 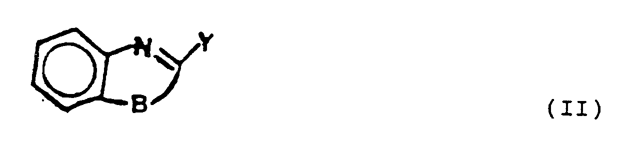 wherein -B- has the meaning set forth above and wherein Y is a leaving group, with a compound having the formula III

   CN - CH₂ - R³ (III)

   wherein R³ has the meaning set forth above, to form a compound of the invention, or
b) reacting a reactive derivative of a compound having the general formula IV 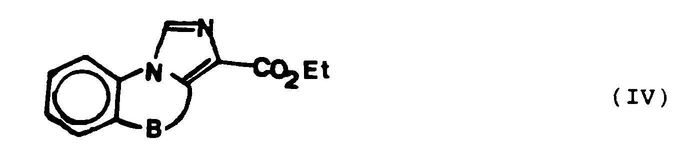 wherein -B- has the meaning set forth above, with a compound having the general formula V

   R'- C(=NOH)NH₂ (V)

   wherein R' has the meaning set forth above to form a compound of the general formula I wherein R³ is 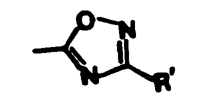 wherein R' has the meaning set forth above, or
c) reacting a compound having the general formula VI 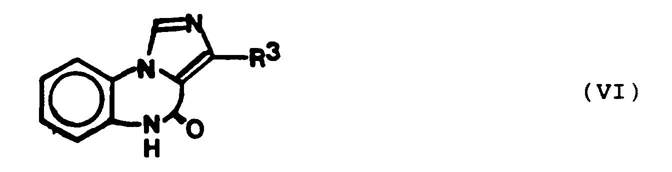 wherein R³ has the meaning set forth above, with POCl₃ to form a compound of formula VII 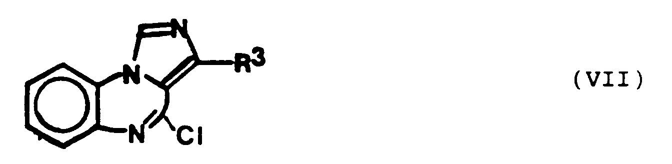 which is reacted with a compound of formula VIII

   HNR''' R'''' (VIII)

   to form a compound of the general formula I wherein B is -N=C(R'')-
   wherein R'' is -NR''' R''''
   wherein R''' and R'''' have the meanings set forth above.

The leaving group, Y, may be any suitable leaving group and, for example, those disclosed in U.S. Patents 4,031,079 or 4,359,420, for example, halogen, alkylthio, e.g., methylthio, aralkylthio, N-nitrosoalkylamino, alkoxy, mercapto, -OP(O)(OR)₂ wherein R is lower-alkyl or -OP(O)(NR 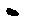 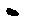 R 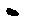 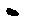 )₂ wherein R 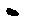 and R 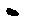 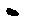 each represents lower-alkyl or phenyl, or together with the nitrogen atom to which they are attached represent a heterocyclic radical such as morpholino, pyrrolidino, piperidino, or methylpiperazino. The reaction is preferably carried out under alkaline conditions, i.e., in the presence of a base, and among bases alkali metal, e.g., potassium or sodium, alkoxides or hydrides are preferred. The reaction is preferably conducted in the presence of an organic solvent which is nonreactive with the reactants and products of reaction under the conditions of reaction, especially an anhydrous solvent and preferably an anhydrous aprotic solvent such as dimethylformamide (DMF) or the like. The temperature range employed may be any range suitable for the reaction to proceed at a reasonable rate and without undue delay or decomposition and a range from a minus forty (-40) degrees Celsius to about room temperature is accordingly usually particularly suitable.

The starting materials may be prepared from commercially available organic compounds and by using well known synthetic methods.

The pharmaceutical properties of the compounds of the invention can be illustrated by determining their capability for displacing radioactive labelled flunitrazepam from benzodiazepine receptors.

The displacement activity of the compounds of the invention may be found by determining the ED₅₀ value. The ED₅₀ value represents the dose (mg/kg) of a test substance which causes the specific binding of flunitrazepam to benzodiazepine receptors in a living brain to be reduced to 50% of the control value.

Such an in vivo test is carried out as described in US 4,774,245.

Test results obtained by testing some compounds of the invention will appear from the following table I.

**TABLE 1**

| Compound | ED₅₀ (mg/kg) |
|---|---|
| 4 | 4.1 |
| 19 | 1.6 |
| 14 | 1.9 |
| 13 | 1.5 |

The compound of the invention, together with a conventional adjuvant, carrier, or diluent, and if desired in the form of a pharmaceutically-acceptable acid addition salt thereof, may be placed into the form of pharmaceutical compositions and unit dosages thereof, and in such form may be employed as solids, such as tablets or filled capsules, or liquids, such as solutions, suspensions, emulsions, elixirs, or capsules filled with the same, all for oral use, in the form of suppositories for rectal administration; or in the form of sterile injectable solutions for parenteral (including subcutaneous) use. Such pharmaceutical compositions and unit dosage forms thereof may comprise conventional ingredients in conventional proportions, with or without additional active compounds or principles, and such unit dosage forms may contain any suitable effective central nervous system ailment alleviating amount of the active ingredient commensurate with the intended daily dosage range to be employed. Tablets containing one (1) milligram of active ingredient or, more broadly, one (1) to thirty (30) milligrams, per tablet, are accordingly suitable representative unit dosage forms.

The compounds of this invention can thus be used for the formulation of pharmaceutical preparations, e.g., for oral and parenteral administration to mammals including humans, in accordance with conventional methods of galenic pharmacy.

Conventional excipients are such pharmaceutically acceptable organic or inorganic carrier substances suitable for parenteral or oral application which do not deleteriously react with the active compound.

Examples of such carriers are water, salt solutions, alcohols, polyethylene glycols, polyhydroxyethoxylated castor oil, gelatin, lactose, amylose, magnesium stearate, talc, silicic acid, fatty acid monoglycerides and diglycerides, pentaerythritol fatty acid esters, hydroxymethylcellulose and polyvinylpyrrolidone.

The pharmaceutical preparations can be sterilized and mixed, if desired, with auxilliary agents, such as lubricants, preservatives, stabilizers, wetting agents, emulsifiers, salt for influencing osmotic pressure, buffers and/or coloring substances and the like, which do not deleteriously react with the active compound.

For parenteral application, particularly suitable are injectable solutions or suspensions, preferably aqueous solutions with the active compound dissolved in polyhydroxylated castor oil.

Ampoules are convenient unit dosage forms.

For oral application, particularly suitable are tablets, dragees, or capsules having talc and/or a carbohydrate carrier or binder or the like, the carrier preferably being lactose and/or corn starch and/or potato starch. A syrup, elixir or like can be used when a sweetened vehicle can be employed. Generally, as to broader ranges, the compounds of the invention are dispensed in unit dosage form comprising 0.05-100 mg in a pharmaceutically-acceptable carrier per unit dosage.

A typical tablet which may be prepared by conventional tabletting techniques contains:

| | |
|---|---|
| Active compound | 1.0 mg |
| Lactosum | 67.8 mg Ph.Eur. |
| Avicel® | 31.4 mg |
| Amberlite® IRP 88 | 1.0 mg |
| Magnesii stearas | 0.25 mg Ph.Eur. |

Due to their high degree of affinity for the benzodiazepin receptors, the compounds of the invention are extremely useful in the treatment of central nervous system ailments or disorders, when administered in an amount effective for the alleviation, amelioration, or elimination thereof. The important CNS activity of the compounds of the invention includes both anticonvulsant and anxiolytic activities along with a low toxicity, together presenting a most favorable therapeutic index. The compounds of the invention may accordingly be administered to a subject, e.g., a living animal or a human body, in need of the same for the treatment, alleviation, amelioration, or elimination of an indication, associated with the central nervous system and the socalled benzodiazepine receptors, which requires such psychopharmaceutical treatment, e.g., especially convulsion, insomnia, dementia and/or anxiety states, if desired in the form of a pharmaceutically acceptable acid addition salt thereof (such as the hydrobromide, hydrochloride, or sulfate, in any event prepared in the usual or conventional manner, e.g., evaporation to dryness of the free base in solution together with the acid), ordinarily concurrently, simultaneously, or together with a pharmaceutically-acceptable carrier or diluent, especially and preferably in the form of a pharmaceutical composition thereof, whether by oral, rectal, or parenteral (including subcutaneous) route, in an effective psychopharmaceutical central nervous system ailment alleviating amount, e.g., an anticonvulsant and/or anxiolytic amount, and in any event an amount which is effective for the alleviation of such a central nervous system ailment due to their benzodiazepine receptor affinity. Suitable dosage ranges are 1-200 milligrams daily, 1-100 milligrams daily, and especially 1-30 milligrams daily, depending as usual upon the exact mode of administration, form in which administered, the indication toward which the administration is directed, the subject involved and the body weight of the subject involved, and the preference and experience of the physician or veterinarian in charge.

The invention will now be described in further detail with reference to the following examples:

### EXAMPLE 1

### Preparation of intermediates having the formula VII 4-chloro-3-(5-cyclopropyl-1,2,4-oxadiazol-3-yl)-imidazo[1,5-a]quinoxaline

A ground mixture of 3-(5-cyclopropyl-1,2,4-oxadiazol-3-yl)-4,5-dihydro-4-oxo-imidazo[1,5-a]quinoxaline (3.76 g, 13 mmol) and phosphorus pentachloride (2.67 g, 13 mmol) in phosphorus oxychloride (10 ml) was stirred for 2 h at 150-160°C. The resulting solution while still warm was poured into 200 ml of ice and stirred for 1 h. The precipitate was collected by filtration, rinsed with water and dried to give 3.4 g of the title compound, m.p. 140-150°C (dec.). The crude product obtained in this way was processed without further purification. (Compound 1).

Similarly, 4-chloro-3-(3-cyclopropyl-1,2,4-oxadiazol-5-yl)-imidazo[1,5-a]quinoxaline, m.p. 166-168°C was prepared from 3-(3-cyclopropyl-1,2,4-oxadiazol-5-yl)-4,5-dihydro-4-oxo-imidazo[1,5-a]quinoxaline. (Compound 2).

### EXAMPLE 2

### 4-Amino-3-(5-cyclopropyl-1,2,4-oxadiazol-3-yl)-imidazo[1,5-a]quinoxaline

A stirred solution of 4-chloro-3-(5-cyclopropyl-1,2,4-oxadiazol-3-yl)-imidazo[1,5-a]quinoxaline (0.15 g) in 10 ml of a 1:1 mixture of CH₂Cl₂ and ethanol was saturated with ammonia. The reaction was monitored by t.l.c. (silica gel / CH₂Cl₂-acetone 4:1) and ammonia was bubbled through the solution in between. When the reaction was completed, the solvent was evaporated in vacuo and the residue was triturated with 10 ml of water. The precipitate was filtered off, rinsed with water and dried to give 0.12 g of the title compound as white needles, m.p. 305-310°C. (Compound 3).

### EXAMPLE 3

### 3-(5-cyclopropyl-1,2,4-oxadiazol-3-yl)-4-dimethylamino-imidazo[1,5-a]quinoxaline hydrochloride

Dimethylamine was bubbled through a solution of 4-chloro-3-(5-cyclopropyl-1,2,4-oxadiazol-3-yl)-imidazo[1,5-a] quinoxaline (3.4 g) in dry THF (75 ml) for 5 min. whereafter the mixture was stirred for 1/2 h. The solvent was evaporated in vacuo and the residue was partioned between CH₂Cl₂ (60 ml) and 1M NaOH (30 ml). The organic layer was washed with water (30 ml) and then shaken with 4 M HCl (60 ml). Pale crystals precipitated and were filtered off and dried to give 2.8 g of the title compound as a dihydrate, m.p. 216-218°C. (Compound 4).

### EXAMPLE 4

### 3-(3-cyclopropyl-1,2,4-oxadiazol-5-yl)-4-dimethylamino-imidazo[1,5-a]quinoxaline.

Dimethylamine was bubbled through a solution of 4-chloro-3-(3-cyclopropyl-1,2,4-oxadiazol-5-yl)-imidazo[1,5-a]quinoxaline (1,5 g) in dry THF for 5 min. After stirring for 1/2 h the solvent was evaporated. The residue was purified by column chromatography (silica gel/ ethyl acetate - benzin 1:1) and the title compound was obtained as pale crystals, yield 0.4 g, m.p. 137-140°C. (Compound 5).

Similarly, from 4-chloro-3-(3-cyclopropyl-1,2,4-oxadiazol-5-yl)-imidazo[1,5-a]quinoxaline and the appropriate amines in THF/triethylamine (10:1) the following compounds were prepared:
3-(3-cyclopropyl-1,2,4-oxadiazol-5-yl)-4-(N-ethyl-N-methylamino)-imidazo[1,5-a]quinoxaline, m.p. 113-115°C. (Compound 6).
3-(3-cyclopropyl-1,2,4-oxadiazol-5-yl)-4-(N-methoxy-N-methylamino)-imidazo[1,5-a]quinoxaline, m.p. 142-144°C. (Compound 7).
3-(3-cyclopropyl-1,2,4-oxadiazol-5-yl)-4-methylamino-imidazo[1,5-a]quinoxaline, m.p. 262-264°C. (Compound 8).

### EXAMPLE 5

### 3-(5-cyclopropyl-1,2,4-oxadiazol-3-yl)-4-morpholino-imidazo[1,5-a]quinoxaline.

A solution of potassium t-butoxide (3.7 g, 32 mmol) in dry DMF (25 ml) was added to a stirred solution of 2-chloro-3-morpholino-quinoxaline (4 g, 16 mmol) and 5-cyclopropyl-3-isocyanomethyl-1,2,4-oxadiazole in dry DMF (50 ml), the temperature being kept at 0-5°C. Then the temperature was raised to 20°C and the solvent was evaporated in vacuo. The residue was partitioned between water (50 ml) and dichloromethane (50 ml). The organic phase was dried and evaporated and the residue was triturated with a small amount of ethyl acetate. The resulting crystalline product was collected by filtration and dried to give 3.2 g of the title compound, m.p. 169-173°C. (Compound 9).

In similar ways the following compounds were prepared:
3-(5-cyclopropyl-1,2,4-oxadiazol-3-yl)-4-(N-ethyl-N-methylamino)-imidazo[1,5-a]quinoxaline hydrochloride, from 2-chloro-3-(N-ethyl-N-methylamino)-quinoxaline and 5-cyclopropyl-3-isocyanomethyl-1,2,4-oxadiazole. The primary product, i.e. the free amine, was obtained as an oil. The hydrochloride, was prepared by dissolving the amine (0.20 g) in dry acetone (10 ml) and adding excess HCl in ether. The resulting precipitate was collected by filtration and dried to give the title compound, m.p. 200-202°C. (Compound 10).
Ethyl 4-(N-ethyl-N-methylamino)-imidazo[1,5-a]quinoxaline-3-carboxylate, an intermediate having the formula IV, m.p. 108-110°C, from 2-chloro-3-(N-ethyl-N-methylamino)-quinoxaline and ethyl isocyanoacetate. (Compound 11).

### EXAMPLE 6

### 3-(3-cyclopropyl-1,2,4-oxadiazol-5-yl)-4-(N-ethyl-N-methylamino)-imidazo[1,5-a]quinoxaline.

A mixture of ethyl 4-(N-ethyl-N-methylamino)-imidazo[1,5-a]quinoxaline-3-carboxylate (1.2 g, 4 mmol), cyclopropanecarboxamidoxime (1.4 g, 14 mmol), crushed 4 Å molecular-sieves (0.5 g), and sodium hydride (0.1 g, 60% in mineral oil) in dry DMF (20 ml) was stirred at ambient temperature for 1 h. Dichloromethane (25 ml) was added and the mixture was filtered through celite. The filtrate was evaporated and the residue was brought to crystallize by the addition of 10 ml of ethyl acetate and cooling to 0°C. The crystals was collected by filtration, rinsed with ethyl acetate and dried to give 0.58 g of the title compound, m.p. 96-97°C. An additional amount (0.22 g) of the product was obtained from the mother liquour. (Compound 6).

### EXAMPLE 7

### Ethyl 5-morpholino-imidazo[1,5-a]quinazoline-3-carboxylate, an intermediate having the formula IV.

A solution of potassium t-butoxide (2.1 g, 19 mmol) in dry DMF (15 ml) was added during 15 min at 0-5°C to a stirred solution of 2-chloro-4-morpholino-quinazoline (3.0 g, 12 mmol) and ethyl isocyanoacetate (2.1 g, 19 mmol) in dry DMF (40 ml). The mixture was stirred at room temperature for 2 h. Then glacial acetic acid (2 ml) was added and the solvent was evaporated in vacuo.

The residue was triturated with a mixture of water (50 ml) and ethyl acetate (10 ml) giving the title compound as a pale yellow precipitate. The product was collected by filtration and rinsed with water and ethyl acetate and dried. Yield 3.6 g (91%), m.p. approx. 165°C, resolidifies to give crystals melting at 195.5 - 196.5°C. (Compound 12).

In a similar manner the following compounds were prepared:
3-(5-cyclopropyl-1,2,4-oxadiazol-3-yl)-5-dimethylamino-imidazo[1,5-a]quinazoline, m.p. 175-176°C, from 2-chloro-4-dimethylamino-quinazoline and 5-cyclopropyl-3-isocyanomethyl-1,2,4-oxadiazole. (Compound 13).
3-(5-cyclopropyl-1,2,4-oxadiazol-3-yl)-5-morpholino-imidazo[1,5-a]quinazoline, m.p. 203-205°C, from 2-chloro-4-morpholino-quinazoline and 5-cyclopropyl-3-isocyanomethyl-1,2,4-oxadiazole. (Compound 14).
3-(5-cyclopropyl-1,2,4-oxadiazol-3-yl)-5-(N-ethyl-N-methylamino)-imidazo[1,5-a]quinazoline, m.p. 161-162°C from 2-chloro-4-(N-ethyl-N-methylamino)-quinazoline and 5-cyclopropyl-3-isocyanomethyl-1,2,4-oxadiazole. (Compound 15).
Ethyl 6-chloro-5-morpholino-imidazo[1,5-a]quinazoline-3-carboxylate, an intermediate of the formula IV being chlorosubstituted in the 6-position, m.p. 189-191°C, from 2,5-dichloro-4-morpholino-quinazoline and ethyl isocyanoacetate. (Compound 16).
3-(5-cyclopropyl-1,2,4-oxadiazol-3-yl)-5-thiomorpholino-imidazo[1,5-a]quinazoline, m.p. 193-196°C, from 2-chloro-4-thiomorpholino-quinazoline and 5-cyclopropyl-3-isocyanomethyl-1,2,4-oxadiazole. (Compound 17).
3-(3-cyclopropyl-1,2,4-oxadiazol-5-yl)-5-thiomorpholino-imidazo[1,5-a]quinazoline, m.p. 228-233 °C, from 2-chloro-4-thiomorpholino-quinazoline and 3-cyclopropyl-5-isocyanomethyl-1,2,4-oxadiazole. (Compound 18).

### EXAMPLE 8

### 3-(3-cyclopropyl-1,2,4-oxadiazol-5-yl)-5-morpholino-imidazo[1,5-a]quinazoline

A mixture of ethyl 5-morpholino-imidazo[1,5-a]quinazoline-3-carboxylate (2.5 g, 7.7 mmol), cyclopropanecarboxamide oxime (3.8 g), crushed 4 Å molecular sieves (7.5 g), and sodium hydride (0.3 g, 60% in mineral oil, 7.7 mmol) in 50 ml of dry DMF was stirred at room temperature for 1 h. Glacial acetic acid (2 ml) and dichloromethane (75 ml) was added, and the mixture was filtered through celite. The filtrate was evaporated and the residue was triturated with water (100 ml). Pale yellow crystals precipitated and was collected by filtration and dried to give 2.3 g of the title compound, m.p. 189-191°C. A pure product was obtained by recrystallization from CH₂Cl₂ / ethyl acetate; yield 1.9 g (69%), m.p. 197-198°C. (Compound 19).

In the same way the following compound was prepared:
6-chloro-3-(3-cyclopropyl-1,2,4-oxadiazol-5-yl)-5-morpholino-imidazo[1,5-a]quinazoline, m.p. 245-246°C, from ethyl 6-chloro-5-morpholino-imidazo[1,5-a]quinazoline-3-carboxylate and cyclopropanecarboxamide oxime. (Compound 20)

## Claims (Claims for the following Contracting State(s): AT, BE, CH, DE, DK, FR, GB, IT, LI, LU, NL, SE)

1. Heterocyclic compounds having the general formula I: 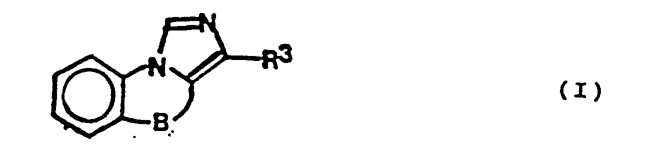 wherein
R³ is 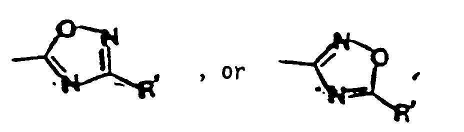 wherein R' is H, C₁₋₆-alkyl or
C₃₋₇-cycloalkyl;
-B- is -C(R'')=N- or -N=C(R'')-
wherein R'' is -NR'''R'''',
wherein R''' and R'''' independently are H, C₁₋₆alkoxy
C₃₋₇-cycloalkyl or C₁₋₆-alkyl and pharmaceutically acceptable acid addition salts thereof.

2. A compound according to claim 1 which is 3-(5-cyclopropyl-1,2,4-oxadiazol-3-yl)-4-dimethylamino-imidazo[1,5-a]quinoxaline hydrochloride.

3. A compound according to claim 1 which is 3-(5-cyclopropyl-1,2,4-oxadiazol-3-yl)-5-dimethylamino-imidazo[1,5-a]quinazoline.

4. A compound according to claim 1 which is 3-(3-cyclopropyl-1,2,4-oxadiazol-5-yl)-5-morpholino-imidazo[1,5-a]quinazoline.

5. A compound according to claim 1 which is 3-(5-cyclopropyl-1,2,4-oxadiazol-3-yl)-5-morpholino-imidazo[1,5-a]quinazoline.

6. A compound according to claim 1 which is 3-(5-cyclopropyl-1,2,4-oxadiazol-3-yl)-4-morpholino-imidazo[1,5-a]quinoxaline.

7. A compound according to claim 1 which is 3-(5-cyclopropyl-1,2,4-oxadiazol-3-yl)-5-thiomorpholino-imidazo[1,5-a]quinazoline.

8. A compound according to claim 1 which is 3-(3-cyclopropyl-1,2,4-oxadiazol-5-yl)-5-thiomorpholino-imidazo[1,5-a]quinazoline.

9. A compound which is 6-chloro-3-(3-cyclopropyl-1,2,4-oxadiazol-5-yl)-5-morpholino-imidazo[1,5-a]quinazoline.

10. A compound which is ethyl 5-morpholino-imidazo[1,5-a]quinazoline-3-carboxylate.

11. A compound which is ethyl-6-chloro-5-morpholino-imidazo[1,5-a] quinazoline-3-carboxylate.

12. A method of preparing a compound according to any one of the claims 1 to 8, **characterized** in
a) reacting a compound of formula II 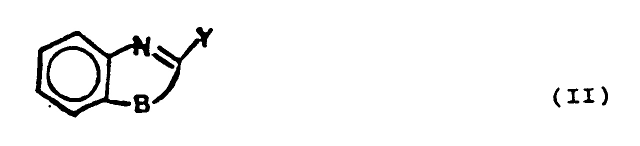 wherein -B- has the meaning set forth above and wherein Y is a leaving group, with a compound having the formula III
CN - CH₂ - R³ (III)
wherein R³ has the meaning set forth above, to form a compound of the invention, or
b) reacting a reactive derivative of a compound having the general formula IV 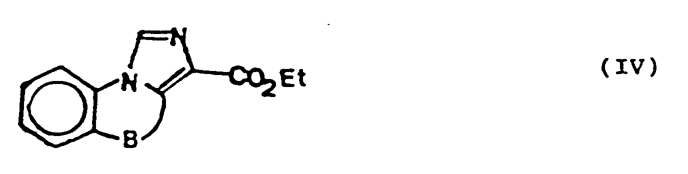 wherein -B- has the meaning set forth above, with a compound having the general formula V
R' - C(=NOH)NH₂ (V)
wherein R' has the meaning set forth above to form a compound of the general formula I wherein R³ is 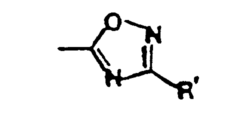 wherein R' has the meaning set forth above, or
c) reacting a compound having the general formula VI 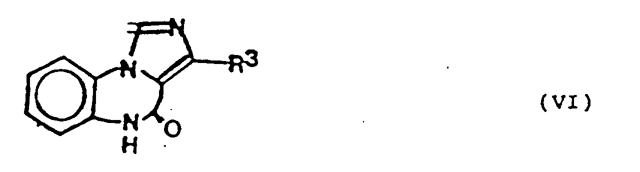 wherein R³ has the meaning set forth above, with POCl₃ to form a compound of formula VII 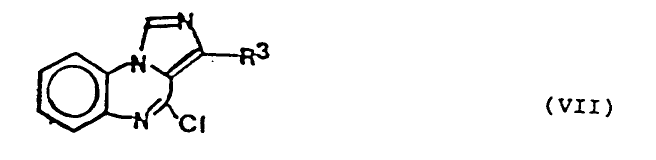 which is reacted with a compound of formula VIII
HNR''' R'''' (VIII)
to form a compound of the general formula I wherein B is -N=C(R'')-
wherein R'' is -NR''' R''''
wherein R''' and R'''' have the meanings set forth above.

13. A pharmaceutical composition comprising an amount of a compound according to any one of claims 1 to 8 together with a pharmaceutically acceptable carrier or diluent.

14. A pharmaceutical composition suitable for use in the treatment of a central nervous system ailment comprising an amount of a compound according to any one of claims 1 to 8 which is effective for the alleviation of such disorder together with a pharmaceutically acceptable carrier or diluent.

15. A pharmaceutical composition according to claim 14 wherein it is in the form of an oral dosage unit containing 1-100 mg of the active compound.

16. Use of a compound according to any one of claims 1 to 8 for preparation of a medicament.

17. Use of a compound according to any one of claims 1 to 8 for preparation of a medicament for the treatment of a central nervous system ailment.

## Claims (Claims for the following Contracting State(s): ES)

1. A method of preparing heterocyclic compounds having the general formula I: 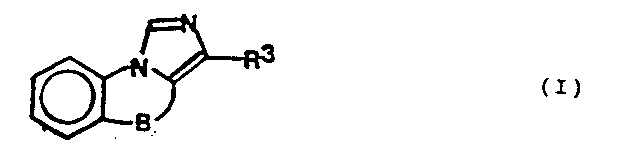 wherein
R³ is 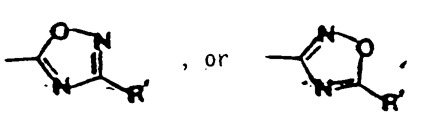 wherein R' is H, C₁₋₆-alkyl or
C₃₋₇-cycloalkyl;
-B- is -C(R'')=N- or -N=C(R'')-
wherein R'' is -NR'''R'''',
wherein R''' and R'''' independently are H, C₁₋₆alkoxy
C₃₋₇-cycloalkyl or C₁₋₆-alkyl and pharmaceutically acceptable acid addition salts thereof, **characterized** in
a) reacting a compound of formula II 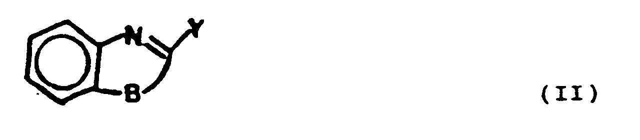 wherein -B- has the meaning set forth above and wherein Y is a leaving group, with a compound having the formula III
CN - CH₂ - R³ (III)
wherein R³ has the meaning set forth above, to form a compound of the invention, or
b) reacting a reactive derivative of a compound having the general formula IV 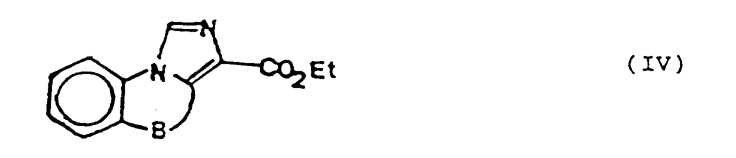 wherein -B- has the meaning set forth above, with a compound having the general formula V
R' - C(=NOH)NH₂ (V)
wherein R' has the meaning set forth above to form a compound of the general formula I wherein R³ is 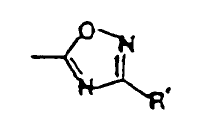 wherein R' has the meaning set forth above, or
c) reacting a compound having the general formula VI 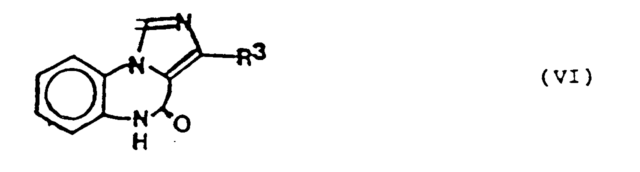 wherein R³ has the meaning set forth above, with POCl₃ to form a compound of formula VII 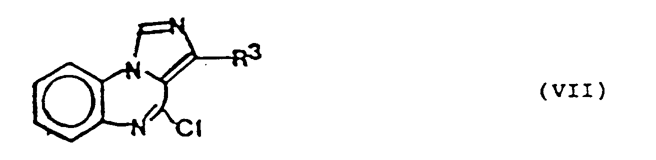 which is reacted with a compound of formula VIII
HNR''' R'''' (VIII)
to form a compound of the general formula I wherein B is -N=C(R'')-
wherein R'' is -NR''' R''''
wherein R''' and R'''' have the meanings set forth above.

2. A method according to claim 1 wherein the compound of formula I is 3-(5-cyclopropyl-1,2,4-oxadiazol-3-yl)-4-dimethylamino-imidazo[1,5-a]quinoxaline hydrochloride.

3. A method according to claim 1 wherein the compound of formula I is 3-(5-cyclopropyl-1,2,4-oxadiazol-3-yl)-5-dimethylamino-imidazo[1,5-a]quinazoline.

4. A method of preparing a pharmaceutical composition comprising mixing an amount of a compound according to any one of claims 1 to 3 with a pharmaceutically acceptable carrier or diluent.

5. A method according to claim 4 wherein the pharmaceutical composition is in the form of an oral dosage unit containing 1-100 mg of the active compound.

6. Use of a compound according to any one of claims 1 to 3 for preparation of a medicament.

7. Use of a compound according to any one of claims 1 to 3 for preparation of a medicament for the treatment of a central nervous system ailment.

## Claims (Claims for the following Contracting State(s): GR)

1. Heterocyclic compounds having the general formula I: 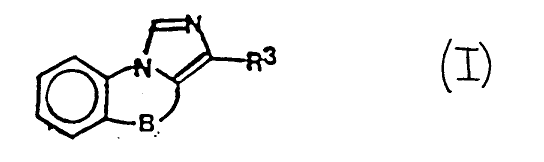 wherein
R³ is 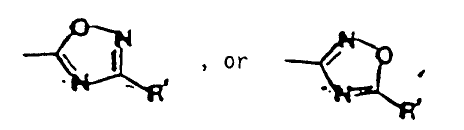 wherein R' is H, C₁₋₆-alkyl or
C₃₋₇-cycloalkyl;
-B- is -C(R'')=N- or -N=C(R'')-
wherein R'' is -NR'''R'''',
wherein R''' and R'''' independently are H, C₁₋₆alkoxy
C₃₋₇-cycloalkyl or C₁₋₆-alkyl and pharmaceutically acceptable acid addition salts thereof.

2. A compound according to claim 1 which is 3-(5-cyclopropyl-1,2,4-oxadiazol-3-yl)-4-dimethylamino-imidazo[1,5-a]quinoxaline hydrochloride.

3. A compound according to claim 1 which is 3-(5-cyclopropyl-1,2,4-oxadiazol-3-yl)-5-dimethylamino-imidazo[1,5-a]quinazoline.

4. A compound according to claim 1 which is 3-(3-cyclopropyl-1,2,4-oxadiazol-5-yl)-5-morpholino-imidazo[1,5-a]quinazoline.

5. A compound according to claim 1 which is 3-(5-cyclopropyl-1,2,4-oxadiazol-3-yl)-5-morpholino-imidazo[1,5-a]quinazoline.

6. A compound according to claim 1 which is 3-(5-cyclopropyl-1,2,4-oxadiazol-3-yl)-4-morpholino-imidazo[1,5-a]quinoxaline.

7. A compound according to claim 1 which is 3-(5-cyclopropyl-1,2,4-oxadiazol-3-yl)-5-thiomorpholino-imidazo[1,5-a]quinazoline.

8. A compound according to claim 1 which is 3-(3-cyclopropyl-1,2,4-oxadiazol-5-yl)-5-thiomorpholino-imidazo[1,5-a]quinazoline.

9. A compound which is 6-chloro-3-(3-cyclopropyl-1,2,4-oxadiazol-5-yl)-5-morpholino-imidazo[1,5-a]quinazoline.

10. A compound which is ethyl 5-morpholino-imidazo[1,5-a]quinazoline-3-carboxylate.

11. A compound which is ethyl-6-chloro-5-morpholino-imidazo[1,5-a] quinazoline-3-carboxylate.

12. A method of preparing a compound according to any one of the claims 1 to 8, **characterized** in
a) reacting a compound of formula II 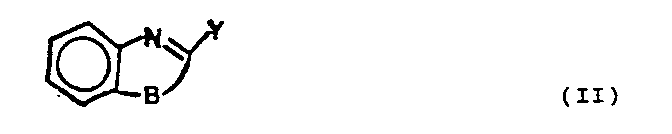 wherein -B- has the meaning set forth above and wherein Y is a leaving group, with a compound having the formula III
CN - CH₂ - R³ (III)
wherein R³ has the meaning set forth above, to form a compound of the invention, or
b) reacting a reactive derivative of a compound having the general formula IV 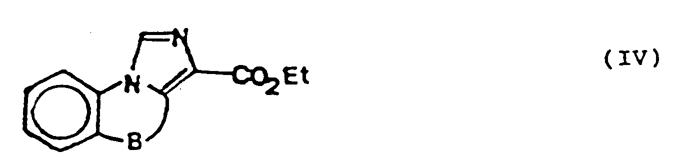 wherein -B- has the meaning set forth above, with a compound having the general formula V
R' - C(=NOH)NH₂ (V)
wherein R' has the meaning set forth above to form a compound of the general formula I wherein R³ is 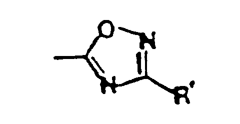 wherein R' has the meaning set forth above, or
c) reacting a compound having the general formula VI 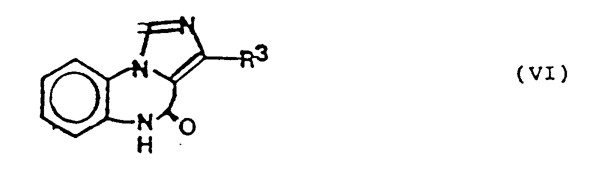 wherein R³ has the meaning set forth above, with POCl₃ to form a compound of formula VII 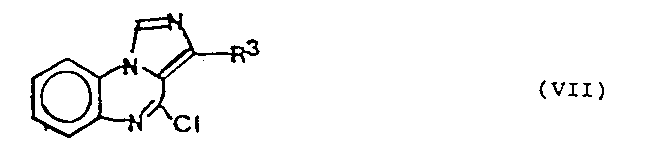 which is reacted with a compound of formula VIII
HNR''' R'''' (VIII)
to form a compound of the general formula I wherein B is -N=C(R'')-
wherein R'' is -NR''' R''''
wherein R''' and R'''' have the meanings set forth above.

13. A method of preparing a pharmaceutical composition comprising mixing an amount of a compound according to any one of claims 1 to 8 together with a pharmaceutically acceptable carrier or diluent.

14. A method according to claim 13 wherein the pharmaceutical composition is in the form of an oral dosage unit containing 1-100 mg of the active compound.

15. Use of a compound according to any one of claims 1 to 8 for preparation of a medicament.

16. Use of a compound according to any one of claims 1 to 8 for preparation of a medicament for the treatment of a central nervous system ailment.

## Patentansprüche (Patentansprüche für folgende(n) Vertragsstaat(en): AT, BE, CH, DE, DK, FR, GB, IT, LI, LU, NL, SE)

1. Heterocyclische Verbindungen mit der allgemeinen Formel I: 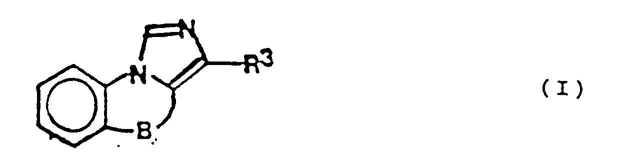 worin R³ die Bedeutung 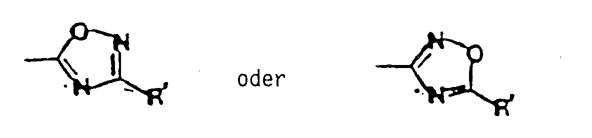 hat, worin R' die Bedeutung H, C₁₋₆-Alkyl oder C₃₋₇-Cycloalkyl hat;
-B- die Bedeutung -C(R'')=N- oder -N=C(R'')- hat,
worin R'' die Bedeutung -NR''' R'''' hat,
worin R''' und R'''' unabhängig die Bedeutung H, C₁₋₆-Alkoxy, C₃₋₇-Cycloalkyl oder C₁₋₆-Alkyl haben, und pharmazeutisch verträgliche Säureadditionssalze davon.

2. Verbindung nach Anspruch 1, wobei es sich um 3-(5-Cyclopropyl-1,2,4-oxadiazol-3-yl)-4-dimethylamino-imidazo-[1,5-a]chinoxalin-hydrochlorid handelt.

3. Verbindung nach Anspruch 1, wobei es sich um 3-(5-Cyclopropyl-1,2,4-oxadiazol-3-yl)-5-dimethylamino-imidazo-[1,5-a]chinazolin handelt.

4. Verbindung nach Anspruch 1, wobei es sich um 3-(3-Cyclopropyl-1,2,4-oxadiazol-5-yl)-5-morpholino-imidazo-[1,5-a]chinazolin handelt.

5. Verbindung nach Anspruch 1, wobei es sich um 3-(5-Cyclopropyl-1,2,4-oxadiazol-3-yl)-5-morpholino-imidazo-[1,5-a]chinazolin handelt.

6. Verbindung nach Anspruch 1, wobei es sich um 3-(5-Cyclopropyl-1,2,4-oxadiazol-3-yl)-4-morpholino-imidazo-[1,5-a]chinoxalin handelt.

7. Verbindung nach Anspruch 1, wobei es sich um 3-(5-Cyclopropyl-1,2,4-oxadiazol-3-yl)-5-thiomorpholino-imidazo-[1,5-a]chinazolin handelt.

8. Verbindung nach Anspruch 1, wobei es sich um 3-(3-Cyclopropyl-1,2,4-oxadiazol-5-yl)-5-thiomorpholino-imidazo-[1,5-a]chinazolin handelt.

9. Verbindung nach Anspruch 1, wobei es sich um 6-Chlor-3-(3-cyclopropyl-1,2,4-oxadiazol-5-yl)-5-morpholino-imidazo-[1,5-a]chinazolin handelt.

10. Verbindung nach Anspruch 1, wobei es sich um Ethyl-5-morpholino-imidazo[1,5-a]chinazolin-3-carboxylat handelt.

11. Verbindung nach Anspruch 1, wobei es sich um Ethyl-6-chlor-5-morpholino-imidazo[1,5-a]chinazolin-3-carboxylat handelt.

12. Verfahren zur Herstellung einer Verbindung nach einem der Ansprüche 1 bis 8, gekennzeichnet durch:
a) Umsetzung einer Verbindung der Formel II 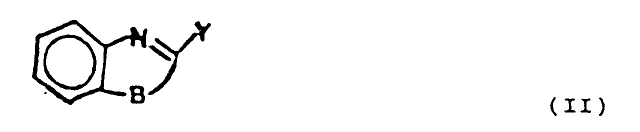 worin -B- die vorstehend angegebene Bedeutung hat und worin Y eine Abgangsgruppe ist, mit einer Verbindung mit der Formel III
CN - CH₂ - R³ (III)
worin R³ die vorstehend angegebene Bedeutung hat, unter Bildung einer erfindungsgemäßen Verbindung, oder
b) Umsetzung eines reaktiven Derivats einer Verbindung mit der allgemeinen Formel IV 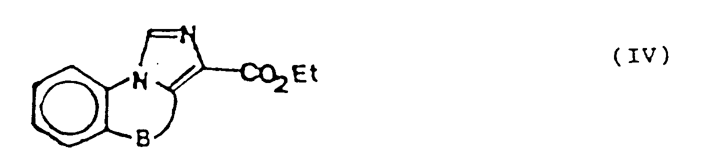 worin -B- die vorstehend angegebene Bedeutung hat, mit einer Verbindung mit der allgemeinen Formel V
R' - C(=NOH)NH₂ (V)
worin R' die vorstehend angegebene Bedeutung hat, unter Bildung einer Verbindung der allgemeinen Formel I, worin R³ die Bedeutung 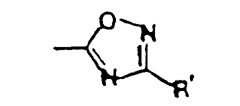 hat, worin R' die vorstehend angegebene Bedeutung hat, oder
c) Umsetzung einer Verbindung mit der allgemeinen Formel VI 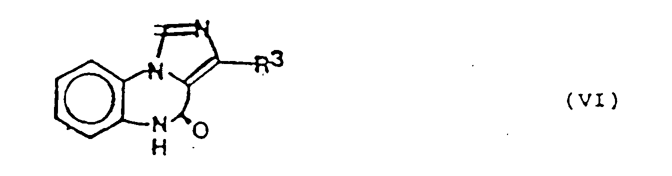 worin R³ die vorstehend angegebene Bedeutung hat, mit POCl₃ unter Bildung einer Verbindung der Formel VII 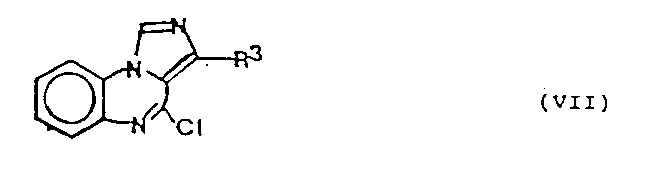 die mit einer Verbindung der Formel VIII
HNR''' R'''' (VIII)
unter Bildung einer Verbindung der allgemeinen Formel I umgesetzt wird, worin B die Bedeutung -N=C(R'')- hat,
worin R'' die Bedeutung -NR''' R'''' hat,
worin R''' und R'''' die vorstehend angegebenen Bedeutungen haben.

13. Pharmazeutische Zusammensetzung, die eine Menge einer Verbindung nach einem der Ansprüche 1 bis 8 zusammen mit einem pharmazeutisch verträglichen Träger oder Verdünnungsmittel umfaßt.

14. Pharmazeutische Zusammensetzung, die sich für die Behandlung von Störungen des zentralen Nervensystems eignet und eine Menge einer Verbindung nach einem der Ansprüche 1 bis 8, die wirksam im Hinblick auf die Linderung einer derartigen Störung ist, zusammen mit einem pharmazeutisch verträglichen Träger oder Verdünnungsmittel umfaßt.

15. Pharmazeutische Zusammensetzung nach Anspruch 14, die in Form einer oralen Dosierungseinheit vorliegt, die 1 bis 100 mg der aktiven Verbindung enthält.

16. Verwendung einer Verbindung nach einem der Ansprüche 1 bis 8 zur Herstellung eines Arzneimittels.

17. Verwendung einer Verbindung nach einem der Ansprüche 1 bis 8 zur Herstellung eines Arzneimittels zur Behandlung einer Störung des zentralen Nervensystems.

## Patentansprüche (Patentansprüche für folgende(n) Vertragsstaat(en): ES)

1. Verfahren zur Herstellung heterocyclischer Verbindungen mit der allgemeinen Formel I: 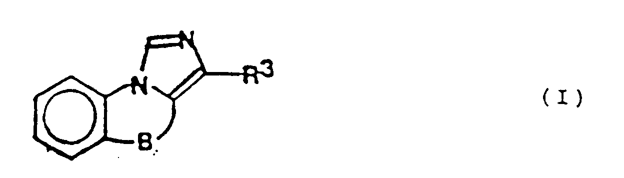 worin R³ die Bedeutung 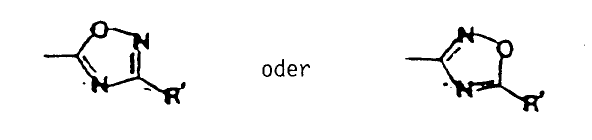 hat, worin R' die Bedeutung H, C₁₋₆-Alkyl oder C₃₋₇-Cycloalkyl hat;
-B- die Bedeutung -C(R'')=N- oder -N=C(R'')- hat,
worin R'' die Bedeutung -NR''' R'''' hat,
worin R''' und R'''' unabhängig die Bedeutung H, C₁₋₆-Alkoxy, C₃₋₇-Cycloalkyl oder C₁₋₆-Alkyl haben, und pharmazeutisch verträglicher Säureadditionssalze davon, gekennzeichnet durch:
a) Umsetzung einer Verbindung der Formel II 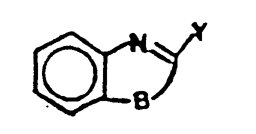 worin -B- die vorstehend angegebene Bedeutung hat und worin Y eine Abgangsgruppe ist, mit einer Verbindung mit der Formel III
CN - CH₂ - R³ (III)
worin R³ die vorstehend angegebene Bedeutung hat, unter Bildung einer erfindungsgemäßen Verbindung, oder
b) Umsetzung eines reaktiven Derivats einer Verbindung mit der allgemeinen Formel IV 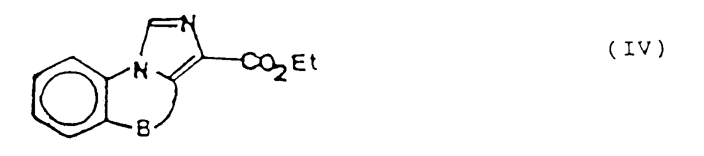 worin -B- die vorstehend angegebene Bedeutung hat, mit einer Verbindung mit der allgemeinen Formel V
R' - C(=NOH)NH₂ (V)
worin R' die vorstehend angegebene Bedeutung hat, unter Bildung einer Verbindung der allgemeinen Formel I, worin R³ die Bedeutung 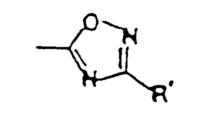 hat, worin R' die vorstehend angegebene Bedeutung hat, oder
c) Umsetzung einer Verbindung mit der allgemeinen Formel VI 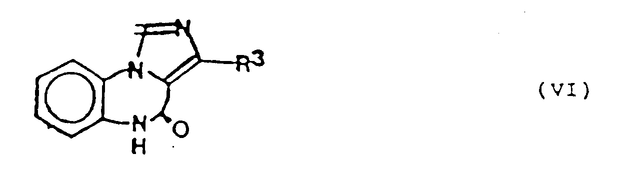 worin R³ die vorstehend angegebene Bedeutung hat, mit POCl₃ unter Bildung einer Verbindung der Formel VII 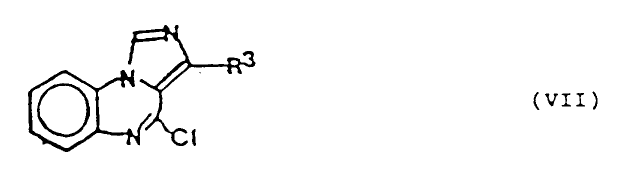 die mit einer Verbindung der Formel VIII
HNR''' R'''' (VIII)
unter Bildung einer Verbindung der allgemeinen Formel I umgesetzt wird, worin B die Bedeutung -N=C(R'')- hat,
worin R'' die Bedeutung -NR''' R'''' hat,
worin R''' und R'''' die vorstehend angegebenen Bedeutungen haben.

2. Verfahren nach Anspruch 1, wobei es sich bei der Verbindung der Formel I um 3-(5-Cyclopropyl-1,2,4-oxadiazol-3-yl)-4-dimethylamino-imidazo-[1,5-a]chinoxalin-hydrochlorid handelt.

3. Verfahren nach Anspruch 1, wobei es sich bei der Verbindung der Formel I um 3-(5-Cyclopropyl-1,2,4-oxadiazol-3-yl)-5-dimethylamino-imidazo-[1,5-a]chinazolin handelt.

4. Verfahren zur Herstellung einer pharmazeutischen Zusammensetzung, umfassend das Mischen einer Menge einer Verbindung nach einem der Ansprüche 1 bis 3 mit einem pharmazeutisch verträglichen Träger oder Verdünnungsmittel.

5. Verfahren nach Anspruch 4, wobei die pharmazeutische Zusammensetzung in der Form einer oralen Dosierungseinheit, die 1 bis 100 mg der aktiven Verbindung enthält, vorliegt.

6. Verwendung einer Verbindung nach einem der Ansprüche 1 bis 3 zur Herstellung eines Arzneimittels.

7. Verwendung einer Verbindung nach einem der Ansprüche 1 bis 3 zur Herstellung eines Arzneimittels zur Behandlung einer Störung des zentralen Nervensystems.

## Patentansprüche (Patentansprüche für folgende(n) Vertragsstaat(en): GR)

1. Heterocyclische Verbindungen mit der allgemeinen Formel I: 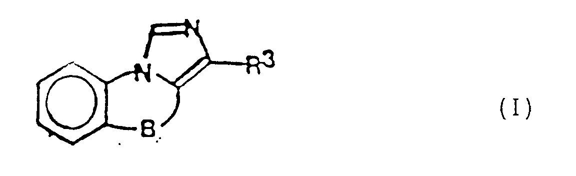 worin R³ die Bedeutung 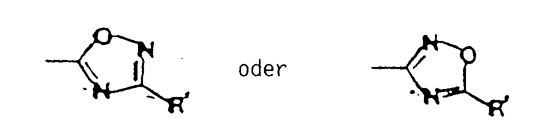 hat, worin R' die Bedeutung H, C₁₋₆-Alkyl oder C₃₋₇-Cycloalkyl hat;
-B- die Bedeutung -C(R'')=N- oder -N=C(R'')- hat,
worin R'' die Bedeutung -NR''' R'''' hat,
worin R''' und R'''' unabhängig die Bedeutung H, C₁₋₆-Alkoxy, C₃₋₇-Cycloalkyl oder C₁₋₆-Alkyl haben, und pharmazeutisch verträgliche Säureadditionssalze davon.

2. Verbindung nach Anspruch 1, wobei es sich um 3-(5-Cyclopropyl-1,2,4-oxadiazol-3-yl)-4-dimethylamino-imidazo-[1,5-a]chinoxalin-hydrochlorid handelt.

3. Verbindung nach Anspruch 1, wobei es sich um 3-(5-Cyclopropyl-1,2,4-oxadiazol-3-yl)-5-dimethylamino-imidazo-[1,5-a]chinazolin handelt.

4. Verbindung nach Anspruch 1, wobei es sich um 3-(3-Cyclopropyl-1,2,4-oxadiazol-5-yl)-5-morpholino-imidazo-[1,5-a]chinazolin handelt.

5. Verbindung nach Anspruch 1, wobei es sich um 3-(5-Cyclopropyl-1,2,4-oxadiazol-3-yl)-5-morpholino-imidazo-[1,5-a]chinazolin handelt.

6. Verbindung nach Anspruch 1, wobei es sich um 3-(5-Cyclopropyl-1,2,4-oxadiazol-3-yl)-4-morpholino-imidazo-[1,5-a]chinoxalin handelt.

7. Verbindung nach Anspruch 1, wobei es sich um 3-(5-Cyclopropyl-1,2,4-oxadiazol-3-yl)-5-thiomorpholino-imidazo-[1,5-a]chinazolin handelt.

8. Verbindung nach Anspruch 1, wobei es sich um 3-(3-Cyclopropyl-1,2,4-oxadiazol-5-yl)-5-thiomorpholino-imidazo-[1,5-a]chinazolin handelt.

9. Verbindung nach Anspruch 1, wobei es sich um 6-Chlor-3-(3-cyclopropyl-1,2,4-oxadiazol-5-yl)-5-morpholino-imidazo-[1,5-a]chinazolin handelt.

10. Verbindung nach Anspruch 1, wobei es sich um Ethyl-5-morpholino-imidazo[1,5-a]chinazolin-3-carboxylat handelt.

11. Verbindung nach Anspruch 1, wobei es sich um Ethyl-6-chlor-5-morpholino-imidazo[1,5-a]chinazolin-3-carboxylat handelt.

12. Verfahren zur Herstellung einer Verbindung nach einem der Ansprüche 1 bis 8, gekennzeichnet durch:
a) Umsetzung einer Verbindung der Formel II 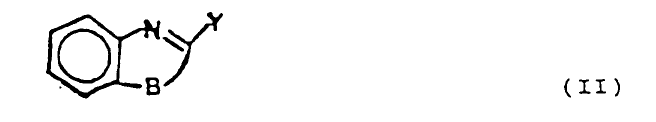 worin -B- die vorstehend angegebene Bedeutung hat und worin Y eine Abgangsgruppe ist, mit einer Verbindung mit der Formel III
CN - CH₂ - R³ (III)
worin R³ die vorstehend angegebene Bedeutung hat, unter Bildung einer erfindungsgemäßen Verbindung, oder
b) Umsetzung eines reaktiven Derivats einer Verbindung mit der allgemeinen Formel IV 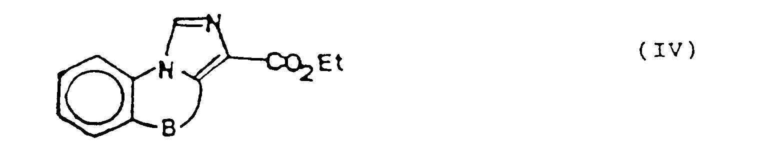 worin -B- die vorstehend angegebene Bedeutung hat, mit einer Verbindung mit der allgemeinen Formel V
R' - C(=NOH)NH₂ (V)
worin R' die vorstehend angegebene Bedeutung hat, unter Bildung einer Verbindung der allgemeinen Formel I, worin R³ die Bedeutung 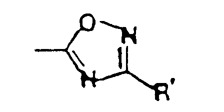 hat, worin R' die vorstehend angegebene Bedeutung hat, oder
c) Umsetzung einer Verbindung mit der allgemeinen Formel VI 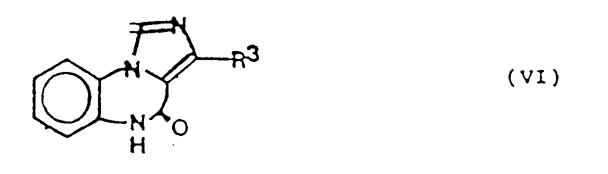 worin R³ die vorstehend angegebene Bedeutung hat, mit POCl₃ unter Bildung einer Verbindung der Formel VII 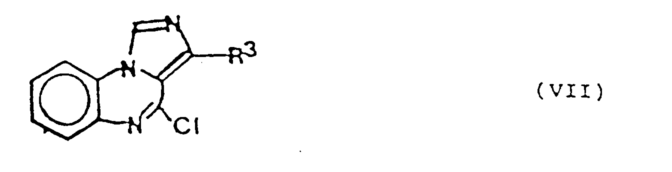 die mit einer Verbindung der Formel VIII
HNR''' R'''' (VIII)
unter Bildung einer Verbindung der allgemeinen Formel I umgesetzt wird, worin B die Bedeutung -N=C(R'')- hat,
worin R'' die Bedeutung -NR''' R'''' hat,
worin R''' und R'''' die vorstehend angegebenen Bedeutungen haben.

13. Verfahren zur Herstellung einer pharmazeutischen Zusammensetzung, umfassend das Mischen einer Menge einer Verbindung nach einem der Ansprüche 1 bis 8 mit einem pharmazeutisch verträglichen Träger oder Verdünnungsmittel.

14. Verfahren nach Anspruch 13, wobei die pharmazeutische Zusammensetzung in der Form einer oralen Dosierungseinheit, die 1 bis 100 mg der aktiven Verbindung enthält, vorliegt.

15. Verwendung einer Verbindung nach einem der Ansprüche 1 bis 8 zur Herstellung eines Arzneimittels.

16. Verwendung einer Verbindung nach einem der Ansprüche 1 bis 8 zur Herstellung eines Arzneimittels zur Behandlung einer Störung des zentralen Nervensystems.

## Revendications (Revendications pour l'(les) Etat(s) contractant(s) suivant(s): AT, BE, CH, DE, DK, FR, GB, IT, LI, LU, NL, SE)

1. Composés hétérocycliques répondant à la formule générale I: 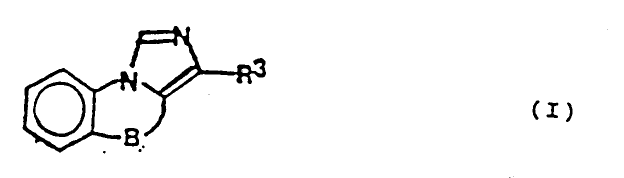 où
R³ est 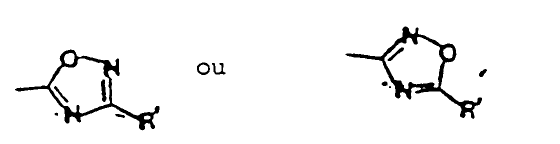 où R' est H, un radical alkyle en C₁₋₆ ou un radical cycloalkyle en C₃₋₇.
-B- est -C(R'')=N- ou -N=C(R'')-
où R'' est -NR'''R'''',
où R''' et R'''' sont indépendamment H, un radical alcoxy en C₁₋₆, un radical cycloalkyle en C₃₋₇ ou un radical alkyle en C₁₋₆ et leurs sels d'addition acide acceptables du point de vue pharmaceutique.

2. Un composé selon la revendication 1, qui est le chlorhydrate de 3-(5-cyclopropyl-1,2,4-oxadiazol-3-yl)-4-diméthylamino-imidazo[1,5,a]quinoxaline.

3. Un composé selon la revendication 1, qui est la 3-(5-cyclopropyl-1,2,4-oxadiazol-3-yl)-5-diméthylamino-imidazo[1,5,a]quinazoline.

4. Un composé selon la revendication 1, qui est la 3-(3-cyclopropyl-1,2,4-oxadiazol-5-yl)-5-morpholino-imidazo [1,5,a]quinazoline.

5. Un composé selon la revendication 1, qui est la 3-(5-cyclopropyl-1,2,4-oxadiazol-3-yl)-5-morpholino-imidazo [1,5,a]quinazoline.

6. Un composé selon la revendication 1, qui est la 3-(5-cyclopropyl-1,2,4-oxadiazol-3-yl)-4-morpholino-imidazo [1,5,a]quinoxaline.

7. Un composé selon la revendication 1, qui est la 3-(5-cyclopropyl-1,2,4-oxadiazol-3-yl)-5-thiomorpholino-imidazo[1,5,a]quinazoline.

8. Un composé selon la revendication 1, qui est la 3-(3-cyclopropyl-1,2,4-oxadiazol-5-yl)-5-thiomorpholino-imidazo[1,5,a]quinazoline.

9. Un composé selon la revendication 1, qui est la 6-chloro-3-(3-cyclopropyl-1,2,4-oxadiazol-5-yl)-5-morpholino-imidazo[1,5,a]quinazoline.

10. Un composé qui est le 3-carboxylate d'éthyl-5-morpholino-imidazo[1,5-a]quinazoline.

11. Un composé qui est le 3-carboxylate d'éthyl-6-chloro-5-morpholino-imidazo[1,5,a]quinazoline.

12. Un procédé de préparation d'un composé selon l'une quelconque des revendications 1 à 8, caractérisé en ce qu'il consiste:
a) à faire réagir un composé de formule II 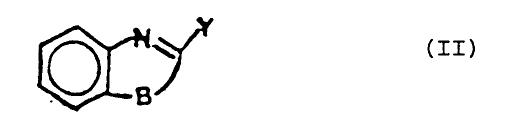 où -B- a la signification mentionnée ci-dessus et où Y est un groupe qui part avec un composé répondant à la formule III
CN - CH₂ - R³ (III)
où R³ a la signification mentionnée ci-dessus pour former un composé de l'invention, ou
b) à faire réagir un dérivé réactif d'un composé répondant à la formule générale IV 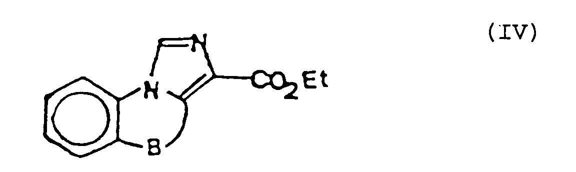 où -B- a la signification mentionnée ci-dessus avec un composé répondant à la formule générale V
R' - C(=NOH)NH₂ (V)
où R' a la signification mentionnée ci-dessus pour former un composé de formule générale I où R³ est 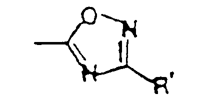 où R' a la signification ci-dessus, ou
c) à faire réagir un composé répondant à la formule générale VI 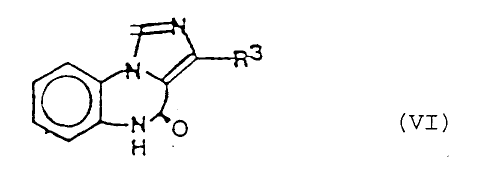 où R³ a la signification mentionnée plus haut avec POCl₃ pour former un composé de formule VII 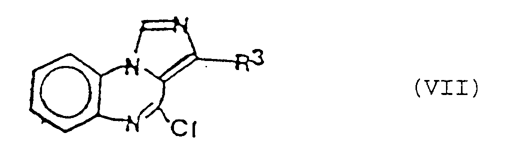 qui est amené à réagir avec un composé de formule VIII
HNR''' R'''' (VIII)
pour former un composé de formule générale I où B est -N=C(R'')-
où R'' est -NR''' R''''
où R''' et R'''' ont les significations mentionnées plus haut.

13. Une composition pharmaceutique comprenant une quantité d'un composé selon l'une quelconque des revendications 1 à 8 conjointement avec un véhicule ou diluant acceptable du point de vue pharmaceutique.

14. Une composition pharmaceutique appropriée pour être utilisée dans le traitement d'une maladie du système nerveux central comprenant une quantité d'un composé selon l'une quelconque des revendications 1 à 8 qui est efficace pour supprimer un tel désordre conjointement avec un véhicule ou diluant acceptable du point de vue pharmaceutique.

15. Une composition pharmaceutique selon la revendication 14 dans laquelle ladite composition est sous la forme d'une unité de dose orale contenant 1 à 100 mg du composé actif.

16. Utilisation d'un composé selon l'une quelconque des revendications 1 à 8 pour la préparation d'un médicament.

17. Utilisation d'un composé selon l'une quelconque des revendications 1 à 8 pour la préparation d'un médicament pour le traitement d'une maladie du système nerveux central.

## Revendications (Revendications pour l'(les) Etat(s) contractant(s) suivant(s): ES)

1. Un procédé de préparation de composés hétérocycliques répondant à la formule générale I: 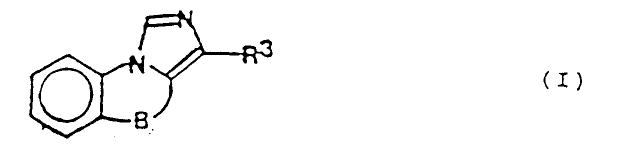 où
R³ est 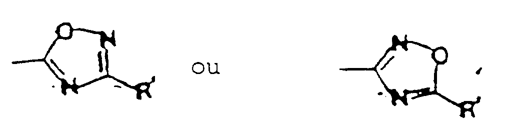 où R' est H, un radical alkyle en C₁₋₆ ou un radical cycloalkyle en C₃₋₇.
-B- est -C(R'')=N- ou -N=C(R'')-
où R'' est -NR'''R'''',
où R''' et R'''' sont indépendamment H, un radical alcoxy en C₁₋₆, un radical cycloalkyle en C₃₋₇ ou un radical alkyle en C₁₋₆ et leurs sels d'addition acide acceptables du point de vue pharmaceutique, caractérisé en ce qu'il consiste:
a) à faire réagir un composé de formule II 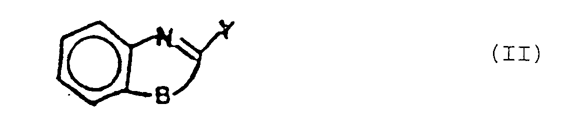 où -B- a la signification mentionnée ci-dessus et où Y est un groupe qui part avec un composé répondant à la formule III
CN - CH₂ - R³ (III)
où R³ a la signification mentionnée ci-dessus pour former un composé de l'invention, ou
b) à faire réagir un dérivé réactif d'un composé répondant à la formule générale IV 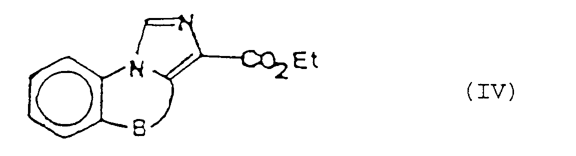 où -B- a la signification mentionnée ci-dessus avec un composé répondant à la formule générale V
R' - C(=NOH)NH₂ (V)
où R' a la signification mentionnée ci-dessus pour former un composé de formule générale I où R³ est 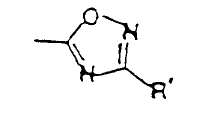 où R' a la signification mentionnée ci-dessus, ou
c) à faire réagir un composé répondant à la formule générale VI 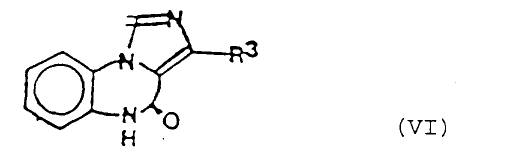 où R³ a la signification mentionnée plus haut avec POCl₃ pour former un composé de formule VII 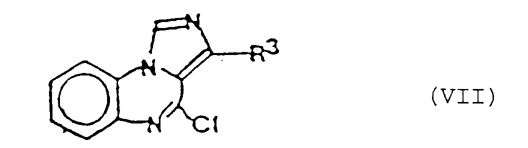 qui est amené à réagir avec un composé de formule VIII
HNR''' R'''' (VIII)
pour former un composé de formule générale I où B est -N=C(R'')-
où R'' est -NR''' R''''
où R''' et R'''' ont les significations mentionnées plus haut.

2. Un procédé selon la revendication 1 dans lequel le composé de formule I est le chlorhydrate de 3-(5-cyclopropyl-1,2,4-oxadiazol-3-yl)-4-diméthylamino-imidazo [1,5,a]quinoxaline.

3. Un procédé selon la revendication 1 dans lequel le composé de formule I est la 3-(5-cyclopropyl-1,2,4-oxadiazol-3-yl)-5-diméthylamino-imidazo[1,5,a]quinazoline.

4. Un procédé de préparation d'une composition pharmaceutique comprenant le mélange d'une quantité d'un composé selon l'une quelconque des revendications 1 à 3 avec un véhicule ou diluant acceptable du point de vue pharmaceutique.

5. Un procédé selon la revendication 4, dans lequel la composition pharmaceutique est sous la forme d'une unité de dose orale contenant 1 à 100 mg du composé actif.

6. Utilisation d'un composé selon l'une quelconque des revendications 1 à 3 pour la préparation d'un médicament.

7. Utilisation d'un composé selon l'une quelconque des revendications 1 à 3 pour la préparation d'un médicament pour le traitement d'une maladie du système nerveux central.

## Revendications (Revendications pour l'(les) Etat(s) contractant(s) suivant(s): GR)

1. Composés hétérocycliques répondant à la formule générale I: 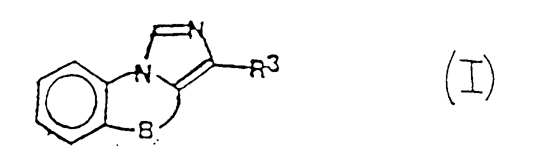 où
R³ est 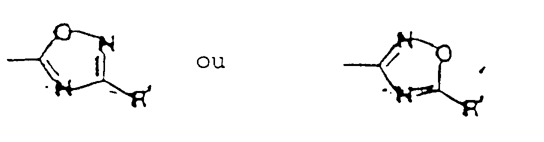 où R' est H, un radical alkyle en C₁₋₆ ou un radical cycloalkyle en C₃₋₇.
-B- est -C(R'')=N- ou -N=C(R'')-
où R'' est -NR'''R'''',
où R''' et R'''' sont indépendamment H, un radical alcoxy en C₁₋₆, un radical cycloalkyle en C₃₋₇ ou un radical alkyle en C₁₋₆ et leurs sels d'addition acide acceptables du point de vue pharmaceutique.

2. Un composé selon la revendication 1, qui est le chlorhydrate de 3-(5-cyclopropyl-1,2,4-oxadiazol-3-yl)-4-diméthylamino-imidazo[1,5,a]quinoxaline.

3. Un composé selon la revendication 1, qui est la 3-(5-cyclopropyl-1,2,4-oxadiazol-3-yl)-5-diméthylamino-imidazo[1,5,a]quinazoline.

4. Un composé selon la revendication 1, qui est la 3-(3-cyclopropyl-1,2,4-oxadiazol-5-yl)-5-morpholino-imidazo [1,5,a]quinazoline.

5. Un composé selon la revendication 1, qui est la 3-(5-cyclopropyl-1,2,4-oxadiazol-3-yl)-5-morpholino-imidazo [1,5,a]quinazoline.

6. Un composé selon la revendication 1, qui est la 3-(5-cyclopropyl-1,2,4-oxadiazol-3-yl)-4-morpholino-imidazo [1,5,a]quinoxaline.

7. Un composé selon la revendication 1, qui est la 3-(5-cyclopropyl-1,2,4-oxadiazol-3-yl)-5-thiomorpholino-imidazo[1,5,a]quinazoline.

8. Un composé selon la revendication 1, qui est la 3-(3-cyclopropyl-1,2,4-oxadiazol-5-yl)-5-thiomorpholino-imidazo[1,5,a]quinazoline.

9. Un composé selon la revendication 1, qui est la 6-chloro-3-(3-cyclopropyl-1,2,4-oxadiazol-5-yl)-5-morpholino-imidazo[1,5,a]quinazoline.

10. Un composé qui est le 3-carboxylate d'éthyl-5-morpholino-imidazo[1,5-a]quinazoline.

11. Un composé qui est le 3-carboxylate d'éthyl-6-chloro-5-morpholino-imidazo[1,5,a]quinazoline.

12. Un procédé de préparation d'un composé selon l'une quelconque des revendications 1 à 8, caractérisé en ce qu'il consiste:
a) à faire réagir un composé de formule II 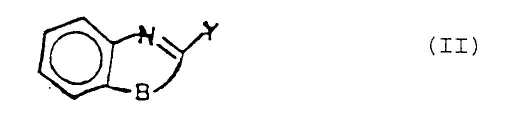 où -B- a la signification mentionnée ci-dessus et où Y est un groupe qui part avec un composé répondant à la formule III
CN - CH₂ - R³ (III)
où R³ a la signification mentionnée ci-dessus pour former un composé de l'invention, ou
b) à faire réagir un dérivé réactif d'un composé répondant à la formule générale IV 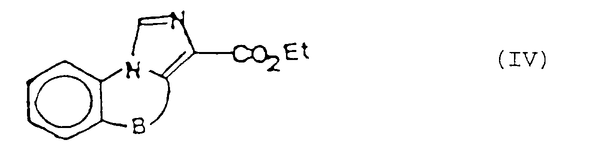 où -B- a la signification mentionnée ci-dessus avec un composé répondant à la formule générale V
R' - C(=NOH)NH₂ (V)
où R' a la signification mentionnée ci-dessus pour former un composé de formule générale I où R³ est 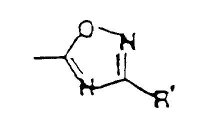 où R' a la signification mentionnée ci-dessus, ou
c) à faire réagir un composé répondant à la formule générale VI 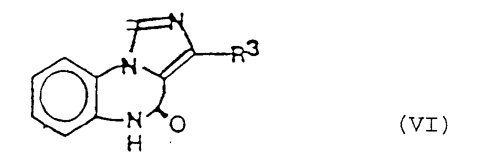 où R³ a la signification mentionnée plus haut avec POCl₃ pour former un composé de formule VII 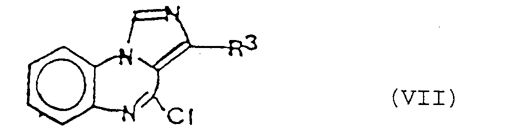 qui est amené à réagir avec un composé de formule VIII
HNR''' R'''' (VIII)
pour former un composé de formule générale I où B est -N=C(R'')-
où R'' est -NR''' R''''
où R''' et R'''' ont les significations mentionnées plus haut.

13. Un procédé de préparation d'une composition pharmaceutique comprenant le mélange d'une quantité d'un composé selon l'une quelconque des revendications 1 à 8 conjointement avec un véhicule ou diluant acceptable du point de vue pharmaceutique.

14. Un procédé selon la revendication 13, dans lequel la composition pharmaceutique est sous la forme d'une unité de dose orale contenant 1 à 100 mg du composé actif.

15. Utilisation d'un composé selon l'une quelconque des revendications 1 à 8 pour la préparation d'un médicament.

16. Utilisation d'un composé selon l'une quelconque des revendications 1 à 8 pour la préparation d'un médicament pour le traitement d'une maladie du système nerveux central.
